# EUROPEAN PATENT APPLICATION

(11) **EP 2 537 545 A1**
(43) Date of publication of application: **26.12.2012**
(21) Application number: 11171155.2
(22) Date of filing: 23.06.2011
(51) Int. Cl.: A61M 5/142, A61M 5/168

(54) **Vented reservoir for medical pump**

(71) Applicant: Debiotech S.A., 1004 Lausanne (CH)
(72) Inventor: Chappel, Eric, 01210 Versonnex (FR); Allendes, Ricardo, 1004 Lausanne (CH); Neftel, Frédéric, 1005 Lausanne (CH)
(74) Representative: Grosfillier, Philippe

(57) **Abstract**

Medical pump comprising a rigid cavity defined between a bottom and a top hard shell (1,3); said cavity comprising an upper face (9), a lower face (10), a lateral face (11) and being divided by a movable membrane (2) which may be moved between said upper and lower faces (9,10), in such a way that a fluid tight reservoir is formed between said top shell (3) and said membrane (2); said bottom shell (1) and or said top shell (3) or both containing several holes (6) which are forming the internal ends of passages (8) communicating between the cavity and the external environment.

## Description

### Field of invention

The present invention is related to medical pumps, and more specifically to reservoirs used with insulin pumps.

### State of the art

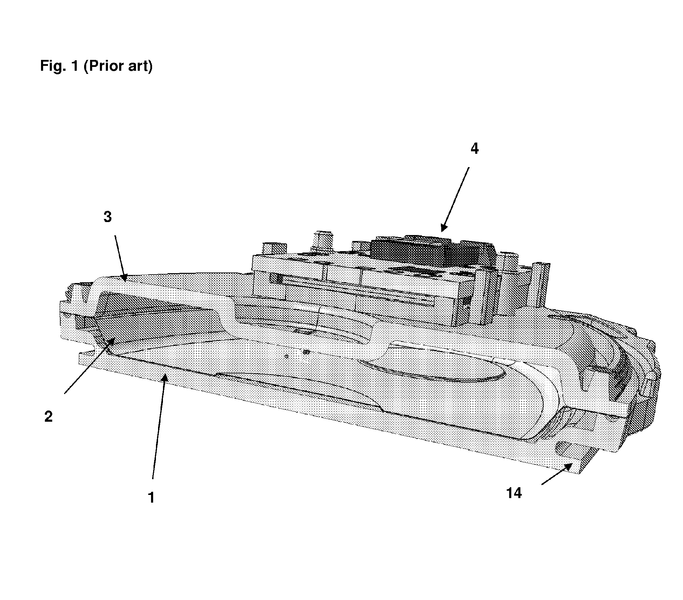

Some insulin pumps, such as the one illustrated on figure 1, have a rigid cavity defined between a top **3** and bottom **1** hard shell. A pump unit **4** is fixed to the top shell **3.** The cavity contains a reservoir which is made of a movable membrane **2** (e.g. soft pouch or flexible film), such as thermoformed and heat-soldered onto the top shell **3** (see for instance international patent application WO 2007/113708). The bottom hard shell **1** protects the membrane **2** against external mechanical forces and ensures a water tightness of the system. The pump is vented using a hydrophobic filter in order to prevent a pressurization of the reservoir due to pressure or temperature changes. The risk of clogging with this filter is high and therefore a potential overdose becomes possible. Moreover such clogging cannot be detected with the gauge pressure detector of the pump because the reference port of the detector is vented by the same filter than the reservoir itself.

The implementation of an additional anti-free-flow valve is a possible way to overcome this problem. See WO 2008/029051. However, this may be expensive for a disposable product and may not be totally fail safe.

### General description of the invention

The present invention provides another and advantageous solution to the clogging risk mentioned previously.

To this effect it relates to a medical pump comprising a rigid cavity defined between a top and a bottom hard shell; said cavity comprising an upper face, a lower face, a lateral face and being divided by a movable membrane which may be moved between said upper and lower faces, in such a way that a fluid tight reservoir is formed between said top shell and said membrane; said bottom shell containing several holes which are oriented towards said lateral face and which are forming the internal ends of passages communicating between the cavity and the external environment.

The lateral face, in some cases, can also be formed by the junction between part of the upper face and part of the lower face.

With the present invention the reservoir is completely vented while maintaining the protection against mechanical forces or ingress of solid foreign objects such as sharp tips. The protection against water ingress is not insured for the reservoir part, which is not necessary when using a tight membrane, while water tightness can be maintained for the electronic part which is added on top of the disposable part separately.

In another embodiment, an hydrophobic surface treatment or coating can also be used on and/or around the holes to limit the water ingress.

### Detailed description of the invention

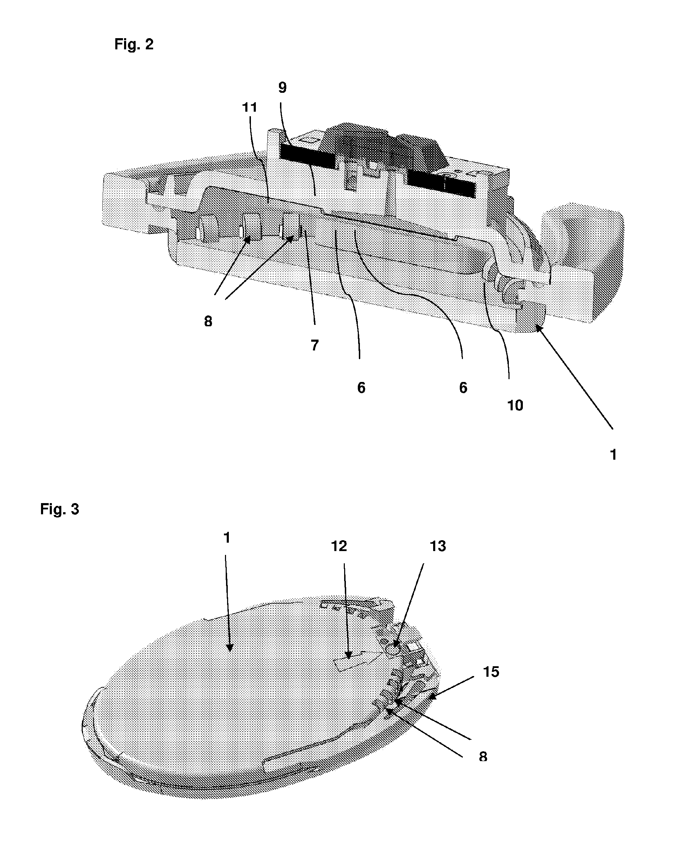

Figure 2 illustrates an embodiment of the invention where the bottom shell **1** is provided with passages **8** on its lateral face **11.** Each passage **8** is provided with a baffle **7** which defines two opposite holes **6** oriented towards the lateral face **11,** in a direction which is parallel with respect to the bottom face.
Figure 3 represents the same embodiment as the one illustrated on figure 2 but viewed from the other side.

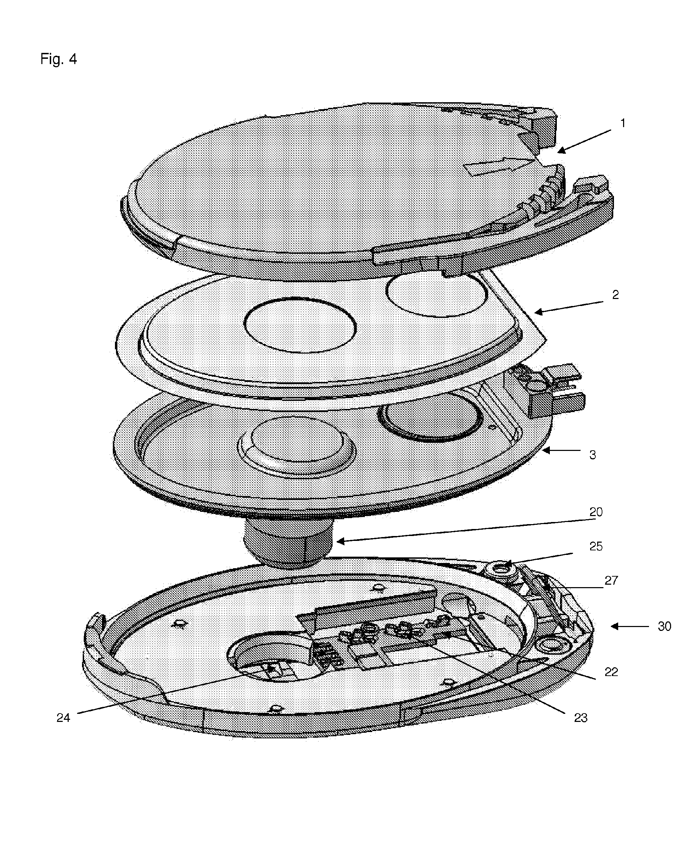

Figure 4 shows an exploded view of the complete system, including the same embodiment as to the one illustrated on Figure 2 and the Pump Controller **30,** the battery **20,** the lock **27,** the Pump Controller vent **25,** the PCB **22** and its spring contacts **23** to connect the pump **4** and finally the battery contact **24.**

In the present invention the design of the bottom shell and more particularly the venting holes are driven by:
● The capability to vent the membrane **2** for any foreseeable use or probable misuse of the pump, including the presence of dirt onto the pump, the wearing of the pump under clothes...
● The protection against solid foreign objects

When several passages **8** are provided in the bottom shell **1** it is not possible to accidentally close all openings because of their specific locations. The compression of the pump against a soft material on the top shell cannot typically obstruct these passages because of their lateral orientated location. The closure of the passages by lateral compression is also prevented by baffles **7** that limit the access typically to fingers.

The passages **8** may have the shape of a slit or any other shape having one dimension preferably lower than 1 mm..

The passages **8** may also be made into a recess and oriented perpendicularly to the normal of the lateral face 11 in order to prevent the insertion of a straight and rigid tip, the minimum dimension of the opening being preferably no longer limited to 1 mm in this configuration according to this recess.

The bottom shell 1 is preferably transparent; the patient should be able to see any large obstruction due to foods or any sticky stuff and eventually to change the disposable. The bottom shell **1** and or the top shell **3** and or the membrane (2) are preferably made in plastic, and more generally in any material having specific grades compatible with insulin. The use of the same material is desirable for thermowelding. The contact surfaces for gluing or thermowelding between the top and bottom shell should be large enough to withstand reservoir overpressure up to 1 bar and drop test from a height of 1 meter or more.

The membrane material has ideally a low elasticity and a low permeability. The membrane thickness is typically smaller than 100 microns.

The surface of the membrane **2** is ideally larger than the surface of the lower face **10** of the bottom shell to prevent any in-plane stress in the membrane and therefore any effect due to the membrane elasticity.

The bottom shell **1** can advantageously include Moiré pattern. In case of overfilling of the reservoir, when the membrane is directly in contact with the bottom shell, the reservoir pressure would bend the bottom shell and induce changes in the Moiré pattern, giving a visual feedback of overfilling to the patient. The Moiré pattern covers partly the bottom shell **1** surface in order to make possible the observation of bubbles into the reservoir.

The bottom shell may include any means to detect deformation due to static load or a pressurized reservoir (e.g. strain gauges, pressure sensors...).

The passages **8** may be partly or completely covered by a removable and permeable tape that ensures the venting of the reservoir. In case of projection of sticky stuff on the passages **8** the patient can advantageously remove the tape instead of trying to clean up the device or simply changing it. The tape may be made of several sheets that can be removed iteratively. Such air permeable tape may also cover the vent **25** of the Pump Controller.

The bottom shell **1** may include marks **12** that help the patient to find the filling port **13.**

The bottom shell **1** is ideally flat and has lateral slides **14** for patch insertion (clipping) and grips **15** for patch removal (unclipping).

Fluid, e.g. water, can flow through the passages **8** and then in the space between the bottom shell 1 and the membrane **2,** the fluid tightness being only provided to the electronic part **30** of the pump which, among other elements, includes the battery **20.** The electronic or pump controller **30** is water tight but has to be vented in case a zincair battery needing oxygen or a gauge pressure sensors are used. The Pump Controller **30** is tightly assembled using lock **27** or clips or any other means onto the upper face of the Top shell **3,** contacting electrically the pads of the pump via the spring contacts **23** of the Printed Circuit Board **22.**

The pump controller is therefore preferably vented using a hydrophobic and or oleophobic filter **25** (Vent).

The hydrophobic and or oleophobic vent **25** is located on the Pump Controller **30** as shown in Figure 4 or on the top shell 3 or on both. If the vent **25** is located in the top shell, the Pump Controller **30** pressure is equilibrated with external environment via the vent **25** and the passages **8.**

Any clogging of the vent **25** is still possible. The pump **4** includes a gauge pressure sensor to measure the pressure in the fluidic pathway between the pump and the patient, the reference port of the gauge pressure sensor being localized inside the Pump Controller **30.** In case of clogging, the reference port of the gauge pressure sensor could become pressurized while the fluidic patient's pressure is kept constant. The detector signals would show large offsets that would trigger over- or under pressure alarms. There is no longer possibility of silent overpressure leading to a free flow from the reservoir to the patient.

## Claims

1. Medical pump comprising a rigid cavity defined between a bottom and a top hard shell (1,3); said cavity comprising an upper face (9), a lower face (10), a lateral face (11) and being divided by a movable membrane (2) which may be moved between said upper and lower faces (9,10), in such a way that a fluid tight reservoir is formed between said top shell (3) and said membrane (2); said bottom shell (1) and or said top shell (3) or both containing several holes (6) which are forming the internal ends of passages (8) communicating between the cavity and the external environment.

2. Medical pump according to claim 1 wherein said holes (6) are oriented towards said lateral face (11).

3. Medical pump according to claim 1 wherein said holes (6) are located within said lateral face (11).

4. Medical pump according to claim 1 wherein said holes (6) are located within said lower face (10).

5. Medical pump according to claim 2 or 3 wherein said holes (6) are oriented in a direction which is forming an angle above 30° with the main direction of their respective passages (8).

6. Medical pump according to one of the previous claims wherein the internal end of said passages (8) is provided with a baffle (7).

7. Medical pump according to one of the previous claims wherein said bottom shell (1) is transparent.

8. Medical pump according to one of the previous claims wherein said bottom shell (1) includes Moiré pattern.

9. Medical pump according to one of the previous claims wherein a hydrophobic surface treatment or coating is added around the holes (6).

10. Medical pump according to one of the previous claims wherein the passages (8) are protected by a removable and permeable tape.

11. Medical pump according to one of the previous claims wherein the bottom shell (1) includes visual filling port localization marks (13).
